# EUROPEAN PATENT SPECIFICATION

(11) **EP 2 559 423 B1**
(45) Date of publication and mention of the grant of the patent: **07.06.2017**
(21) Application number: 11768810.1
(22) Date of filing: 11.04.2011
(51) Int. Cl.: A61K 8/25, A61K 8/06, A61K 8/36, A61K 8/49, A61K 8/86, A61Q 17/04

(54) **OIL-IN-WATER EMULSION COMPOSITION**
ÖL-IN-WASSER-EMULSIONSVERBINDUNG
COMPOSITION D'ÉMULSION HUILE DANS L'EAU

(30) Priority: 08.04.2011 JP 2011086011; 13.04.2010 JP 2010092260
(43) Date of publication of application: 20.02.2013
(73) Proprietor: SHISEIDO COMPANY, LTD., Tokyo 104-8010 (JP)
(72) Inventor: NAGARE, Yuko, Yokohama-shi, Kanagawa 224-8558 (JP); YAMAGUCHI, Kazuhiro, Yokohama-shi, Kanagawa 224-8558 (JP)
(74) Representative: Henkel, Breuer & Partner
(86) International application number: PCT/JP2011/058977
(87) International publication number: WO 2011/129289

(56) References cited:
- EP-A1- 1 051 963
- EP-A1- 2 127 633
- EP-A1- 2 484 339
- WO-A1-2008/080892
- JP-A- 5 238 924
- JP-A- 9 118 726
- JP-A- 2002 155 189
- JP-A- 2004 091 374
- JP-A- 2004 519 499
- JP-A- 2005 350 367
- JP-A- 2010 047 538
- "Polymer dispersions of encapsulated organic UV filters in Personal Care", IP.COM JOURNAL, IP.COM INC., WEST HENRIETTA, NY, US, 12 January 2009 (2009-01-12), XP013127766, ISSN: 1533-0001
- "Suncare compositions with new cosmetic raw materials", IP.COM JOURNAL, IP.COM INC., WEST HENRIETTA, NY, US, 2 March 2010 (2010-03-02), XP013137210, ISSN: 1533-0001

## Description

### Technical Field

The present invention relates to an oil-in-water emulsion composition, and more particularly, to an oil-in-water emulsion composition containing an oil-soluble ultraviolet absorber in an external phase (water phase), and having a high stability and ultraviolet protection ability.

### Background Art

Sunscreen cosmetics are intended to block ultraviolet rays in the sunlight to protect the skin from adverse effects of ultraviolet rays. The base of the sunscreen cosmetics includes emulsion, lotion and oil bases. Among them, an oil-in-water emulsion base, since it provides a fresh sense of use and can be used for preparing a low SPF to a high SPF product, has been widely used (Non Patent Document 1).

In the meantime, the ultraviolet absorbers to be blended in sunscreen cosmetics are classified into oil-soluble ones and water-soluble ones. To obtain a high protection ability for absorbing ultraviolet rays in the UVA region (wavelength of 320 to 400 nm) and the UVB region (wavelength of 290 to 320 nm), a UVB absorber and a UVA absorber need to be blended in a well-balanced manner.

However, most of the oil-soluble ultraviolet absorbers are less soluble. To dissolve such an ultraviolet absorber, a large amount of high-polarity oil content needs to be blended, with the result that, a fresh sense of use intrinsic to an oil-in-water emulsion was lost, which caused a stability problem in some cases such that an ultraviolet absorber precipitated in an oil phase at a low temperature.

Patent Document 1 describes that a less soluble ultraviolet absorber is encapsulated in spherical polymer particles formed of styrene or the like and made into a spherical powder, with the result that solubility in oil is improved to attain a high content in an oil phase. The ultraviolet absorber is blended in water-in-oil emulsion cosmetics or solid cosmetics and not blended in water phases (external phases) of oil-in-water emulsions.

### Prior Art Publications

### Patent Document

Patent Document 1: JP-A-2009-91307

### Non Patent Document

Non Patent Document 1: "New Cosmetic Science, second edition" edited by Takeo Mitsui, Nanzando Co., Ltd., 2001, 497-504 pages

### Summary of the Invention

### Problem to be solved by the Invention

Therefore, an object of the present invention is to provide an oil-in-water emulsion composition which contains an oil-soluble ultraviolet absorber having a low solubility, and also has excellent in stability.

### Means for solving the Problem

To attain the object, the present invention provides an oil-in-water emulsion composition containing:
(a) an aqueous dispersion of composite particles of a first oil-soluble ultraviolet absorber and an organic polymer contained in an external phase (water phase), wherein the first oil-soluble ultraviolet absorber is bis-ethylhexyloxyphenol methoxyphenyl triazine,
(b) a nonionic surfactant and a fatty acid soap,
(c) a water-swellable clay mineral selected from the group consisting of saponite and bentonite,
(d) a higher fatty acid, and
(e) a second oil-soluble ultraviolet absorber dissolved in an internal phase (oil phase), wherein the second oil-soluble ultraviolet absorber is selected from the group consisting of 2-ethylhexyl paramethoxycinnamate, homosalate, octyl salicylate, oxybenzone, 4-t-butyl-4'-methoxydibenzoylmethane, octyl triazone, methylene bis-benzotriazolyl tetramethylbutyl phenol, 2-hydroxy-4-methoxybenzophenone, dihydroxydimethoxybenzophenone, dihydroxybenzophenone, tetrahydroxybenzophenone, hexyl diethylamino hydroxybenzoyl benzoate, 2-cyano-3,3-diphenylacrylic acid 2'-ethylhexyl ester, polysilicone-15 and drometrizole polysiloxane.

### Effects of the Invention

The oil-in-water emulsion composition of the present invention containing an ultraviolet absorber less soluble in oil in a water phase (external phase) provides an improved stability of the system. Furthermore, the oil-in-water emulsion composition can exert an advantageous effect of improving ultraviolet protection ability, compared to that containing the same ultraviolet absorber in an oil phase (internal phase). Accordingly, the oil-in-water emulsion composition of the present invention is particularly suitable for use as a sunscreen cosmetic providing a fresh sense of use and having an excellent ultraviolet protection ability.

### Brief Description of Drawing

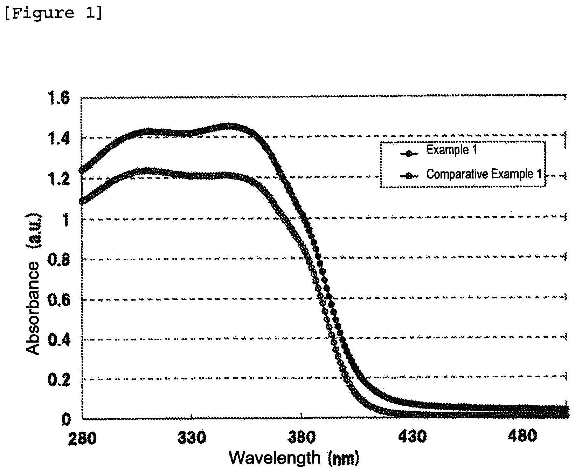

[Figure 1] Figure 1 is a chart showing ultraviolet absorption spectra of the compositions of Example 1 and Comparative Example 1.

### Modes for carrying out the Invention

The oil-in-water emulsion composition of the present invention contains an aqueous dispersion of an oil-soluble ultraviolet absorber (component a) in a water phase (external phase).

The oil-soluble ultraviolet absorber is insoluble in water and less soluble in oil. Substances substantially insoluble in oil, such as methylene bis-benzotriazole tetramethylbutyl phenol, are not included. If an oil-in-water emulsion composition is prepared using an aqueous dispersion of an ultraviolet absorber insoluble in oil and applied to the skin, the resultant skin sometimes looks unnaturally white.

According to the invention, the less soluble ultraviolet absorber is a triazine derivative, namely 2,4-bis-{[4-(2-ethylhexyloxy)-2-hydroxy]phenyl}-6-(4-methoxyphenyl)-(1,3,5)-triazine (hereinafter, referred to as "bis-ethylhexyloxyphenol methoxyphenyl triazine" in the present specification). The bis-ethylhexyloxyphenol methoxyphenyl triazine is commercially available from BASF under the trade name of Tinosorb S, and the commercially available product can be used.

The aqueous dispersion of an oil-soluble ultraviolet absorber as used in the present invention is an aqueous dispersion of composite particles of an oil-soluble ultraviolet absorber and an organic polymer. When a water phase containing the aqueous dispersion is present together with an oil, the incorporation of the oil-soluble ultraviolet absorber into the composite particles suppress the dissolution of the oil-soluble ultraviolet absorber in the water phase into the oil phase.

The aqueous dispersion of composite particles of an oil-soluble ultraviolet absorber and an organic polymer can be prepared, for example, in accordance with a method described in WO2009/007264. In short, emulsion polymerization is performed in the state of dispersing a mixture of an ultraviolet absorber and an organic monomer in water to be able to obtain an aqueous dispersion having composite particles of the ultraviolet absorber and an organic polymer dispersed therein.

As the organic monomer, a monomer having an ethylenic unsaturated bond, such as acrylic acid, methacrylic acid, an alkyl acrylate, an alkyl methacrylate, a styrene monomer and a nylon monomer, is preferably used.

As an aqueous dispersion of such composite particles, a commercially available product from BASF under the trade name of Tinosorb S aqua can be used. Tinosorb S aqua contains composite particles of bis-ethylhexyloxyphenol methoxyphenyl triazine (Tinosorb S) and polymethylmethacrylate (PMMA) dispersed in water. The contents of bis-ethylhexyloxyphenol methoxyphenyl triazine and PMMA are 20 % by mass and 19 % by mass, respectively.

The content of the oil-soluble ultraviolet absorber in the composition of the present invention is 5 % by mass or less, preferably 3 % by mass or less, and more preferably 0.01 to 3 % by mass, on a dry mass basis. If the content is less than 0.01% by mass, a sufficient ultraviolet absorption ability cannot be obtained; whereas, if the content is beyond 5% by mass, it tends to cause a problem in sense of use, such as greasiness.

Note that, provided that an aqueous dispersion (component a) contains 20% by mass of the ultraviolet absorber, the content in terms of the aqueous dispersion is 25 % by mass or less, preferably 15 % by mass or less, and more preferably 0.05 to 15 % by mass.

The composition of the present invention further contains a nonionic surfactant and a fatty acid soap (component b).

As the nonionic surfactants, those having an HLB of 8 or more are preferable. Examples thereof include POE (7) cetyl ether, POE (10) cetyl ether, POE (12) cetyl ether, POE (15) cetyl ether, POE (17) cetyl ether, POE (20) cetyl ether, POE (25) cetyl ether, POE (30) cetyl ether, POE (6) oleyl ether, POE (8) oleyl ether, POE (10) oleyl ether, POE (12) oleyl ether, POE (15) oleyl ether, POE (20) oleyl ether, POE (23) oleyl ether, POE (50) oleyl ether, POE (8) stearyl ether, POE (11) stearyl ether, POE (15) stearyl ether, POE (20) stearyl ether, POE (25) stearyl ether, POE (30) stearyl ether, POE (40) stearyl ether, POE (5) nonyl phenyl ether, POE (10) nonyl phenyl ether, POE (11) nonyl phenyl ether, POE (12) nonyl phenyl ether, POE (13) nonyl phenyl ether, POE (15) nonyl phenyl ether, POE (20) nonyl phenyl ether, POE (25) nonyl phenyl ether, POE (30) nonyl phenyl ether, POE (5) octyl phenyl ether, POE (8) octyl phenyl ether, POE (10) octyl phenyl ether, POE (15) octyl phenyl ether, POE (20) octyl phenyl ether, POE (25) octyl phenyl ether, POE (30) octyl phenyl ether, POE (40) octyl phenyl ether, POE (50) octyl phenyl ether, POE (5) lauryl ether, POE (7) lauryl ether, POE (9) lauryl ether, POE (10) lauryl ether, POE (12) lauryl ether, POE (15) lauryl ether, POE (20) lauryl ether, POE (25) lauryl ether, POE (30) lauryl ether, POE (50) lauryl ether, POE (10) hexyl decyl ether, POE (15) hexyl decyl ether, POE (20) hexyl decyl ether, POE (25) hexyl decyl ether, POE (10) isostearyl ether, POE (15) isostearyl ether, POE (20) isostearyl ether, POE (25) isostearyl ether, POE (10) octyl dodecyl ether, POE (16) octyl dodecyl ether, POE (20) octyl dodecyl ether, POE (25) octyl dodecyl ether, POE (10) decyl pentadecyl ether, POE (20) decyl pentadecyl ether, POE (30) decyl pentadecyl ether, POE (10) decyl tetradecyl ether, POE (15) decyl tetradecyl ether, POE (20) decyl tetradecyl ether, POE (25) decyl tetradecyl ether, POE (10) cholesteryl ether, POE (15) cholesteryl ether, POE (20) cholesteryl ether, POE (24) cholesteryl ether, POE (30) cholesteryl ether, POE (10) lauryl ether stearate, POE (12) dilaurate, POE (16) dilaurate, POE (20) dilaurate, POE (10) monostearate, POE (20) monostearate, POE (30) monostearate, POE (40) monostearate, POE (150) monostearate, POE (150) distearate, POE (6) monoisostearate, POE (12) monoisostearate, POE (20) monoisostearate, POE (6) monooleate, POE (10) monooleate, POE (6) glyceryl monoisostearate, POE (8) glyceryl monoisostearate, POE (10) glyceryl monoisostearate, POE (15) glyceryl monoisostearate, POE (20) glyceryl monoisostearate, POE (25) glyceryl monoisostearate, POE (30) glyceryl monoisostearate, POE (40) glyceryl monoisostearate, POE (50) glyceryl monoisostearate, POE (60) glyceryl monoisostearate, POE (30) glyceryl triisostearate, POE (40) glyceryl triisostearate, POE (50) glyceryl triisostearate, POE (60) glyceryl triisostearate, POE (20) sorbitan monolaurate, POE (20) sorbitan monooleate, POE (40) sorbitan monooleate, POE (30) glyceryl trioleate, POE (40) glyceryl trioleate, POE (50) glyceryl trioleate, POE (60) glyceryl trioleate, POE (5) glyceryl monostearate, POE (10) glyceryl monostearate, POE (15) glyceryl monostearate, POE (20) glyceryl monostearate, POE (30) glyceryl monostearate, POE (40) glyceryl monostearate, POE (60) glyceryl monostearate, POE (20) trimethylolpropane trimyristate, POE (25) trimethylolpropane trimyristate, POE (30) trimethylolpropane trimyristate, POE (25) trimethylolpropane triisostearate, POE (30) trimethylolpropane triisostearate, POE (40) trimethylolpropane triisostearate, POE (50) trimethylolpropane triisostearate, POE (20) hydrogenated castor oil, POE (30) hydrogenated castor oil, POE (40) hydrogenated castor oil, POE (50) hydrogenated castor oil, POE (60) hydrogenated castor oil, POE (80) hydrogenated castor oil, POE (100) hydrogenated castor oil, POE (20) castor oil, POE (30) castor oil, POE (40) castor oil, POE (50) castor oil, POE (20) hydrogenated castor oil monolaurate, POE (30) hydrogenated castor oil monolaurate, POE (40) hydrogenated castor oil monolaurate, POE (50) hydrogenated castor oil monolaurate, POE (60) hydrogenated castor oil monolaurate, POE (30) hydrogenated castor oil monoisostearate, POE (40) hydrogenated castor oil monoisostearate, POE (50) hydrogenated castor oil monoisostearate, POE (60) hydrogenated castor oil monoisostearate, POE (40) hydrogenated castor oil triisostearate, POE (50) hydrogenated castor oil triisostearate, POE (60) hydrogenated castor oil triisostearate, sorbitan monostearate, sorbitan monooleate, sorbitan monoisostearate, self-emulsification type glyceryl monostearate, diglyceryl monostearate, hydrogenated castor oil pyroglutamic acid isostearic acid diester, glyceryl pyroglutamic acid isostearic acid diester, PEG glyceryl isostearate, and PEG glyceryl stearate.

Examples of the fatty acid soaps include potassium laurate, potassium myristate, potassium palmitate, potassium stearate, potassium arachidate, potassium behenate, sodium laurate, sodium myristate, sodium palmitate, sodium stearate, sodium arachidate, sodium behenate, triethanolamine laurate, triethanolamine myristate, triethanolamine palmitate, triethanolamine stearate, triethanolamine arachidate, triethanolamine behenate, aminomethylpropanol laurate, aminomethylpropanol myristate, aminomethylpropanol palmitate, aminomethylpropanol stearate, and aminomethylpropanol arachidate and aminomethylpropanol behenate.

The content of the nonionic surfactant and the fatty acid soap in the composition of the present invention is 10% by mass or less, preferably 5 % by mass or less, and more preferably 0.01 to 3 % by mass. If the content is less than 0.01 % by mass, a stable emulsion is hardly obtained; whereas, if the content is beyond 10% by mass, the resultant product tends to deteriorate in the sense of use, causing greasiness etc.

The composition of the present invention further contains a water-swellable clay mineral (component c). The water-swellable clay mineral (component c) is a type of colloid-containing aluminum silicate having a trilaminar structure.

According to the invention, the water-swellable clay mineral is bentonite or saponite. These may be either one of a natural product and a synthetic product. Examples of commercially available products include Kunipia (manufactured by Kunimine Industries Co., Ltd.) and Smecton (manufactured by Kunimine Industries Co., Ltd.).

The content of the water-swellable clay mineral (component c) is 4 % by mass or less, preferably 2% by mass or less, and more preferably 0.01 to 1% by mass. If the content is less than 0.01 % by mass, stability deteriorates; whereas, if the content is beyond 4 % by mass, the degree of extension tends to be low.

The composition of the present invention further contains a higher fatty acid (component d).

Examples of the higher fatty acid include, but not particularly limited to, lauric acid, myristic acid, palmitic acid, stearic acid, behenic acid, oleic acid, undecylenic acid, tall oil acid, isostearic acid, linoleic acid, linolenic acid, eicosapentaenoic acid (EPA) and docosahexaenoic acid (DHA).

The content of the higher fatty acid (component d) is 10 % by mass or less, preferably 0.1 to 5 % by mass, and more preferably 0.5 to 2 % by mass. If the content is less than 0.1 % by mass, stability deteriorates; whereas, if the content is beyond 10 % by mass, the degree of extension tends to be low.

Furthermore, the stability of the composition of the present invention can be improved by blending a water-soluble polymer.

Examples of the water-soluble polymer include vegetable polymers, microbial polymers and synthetic/semisynthetic polymers. Examples of the vegetable polymers include xanthan gum, Arabian gum, tragacanth gum, galactan, guar gum, carob gum, karaya gum, carrageenan, pectin, agar, quince seed (Cydonia oblonga), algal colloids (brown algae extract), starch (rice, corn, potato, wheat) and glycyrrhizin acid. Examples of the microbial polymers include dextran, succinoglycan and pullulan.

Examples of the semisynthetic water-soluble polymers include starch polymers (e.g., carboxymethyl starch, methylhydroxypropyl starch); cellulose polymers (e.g., methyl cellulose, ethyl cellulose, methylhydroxypropyl cellulose, hydroxyethyl cellulose, sodium cellulose sulfate, hydroxypropyl cellulose, carboxymethyl cellulose, sodium carboxymethyl cellulose, crystalline cellulose, cellulose powder); and alginate polymers (e.g., sodium alginate, propylene glycol alginate).

Examples of synthetic water-soluble polymers include vinyl polymers (e.g., carboxyvinyl polymer (carbomer), polyvinyl alcohol, polyvinyl methyl ether, polyvinyl pyrrolidone); polyoxyethylene polymers (e.g., a polyoxyethylene-polyoxypropylene copolymer of polyethylene glycol 20,000, 40,000, 60,000); acrylic polymers (e.g., polyacrylic acid, polyethyl acrylate, polyacrylamide); polyethylene imines; and cation polymers.

The content of these water-soluble polymers is 3 % by mass or less, preferably 0.5 % by mass or less, and more preferably 0.01 to 0.3 % by mass. If the content is less than 0.01 % by mass, stability deteriorates; whereas if the content is beyond 3 % by mass, the resultant product tends to deteriorate in sense of use, causing greasiness etc.

The oil except a higher fatty acid that can constitute the oil-in-water emulsion composition of the present invention is not particularly limited. Examples of a liquid fat and oil include avocado oil, camellia oil, turtle oil, macadamia nut oil, corn oil, mink oil, olive oil, rapeseed oil, egg yolk oil, sesame oil, persic oil, wheat germ oil, sasanqua oil, castor oil, linseed oil, safflower oil, cottonseed oil, perilla oil, bean oil, peanut oil, tea seed oil, Japanese nutmeg oil, rice bran oil, China wood oil, Japanese tung oil, jojoba oil, germ oil and triglycerin.

Examples of a solid fat and oil include cacao butter, coconut oil, horse fat, hardened coconut oil, palm oil, beef tallow, mutton tallow, hardened beef tallow, palm kernel oil, lard, beef bone fat, Japan wax kernel oil, hardened oil, cow leg fat, Japan wax and hydrogenated castor oil.

Examples of a wax include beeswax, candelilla wax, cotton wax, carnauba wax, bayberry wax, Ibota wax, whale wax, montan wax, rice bran wax, lanolin, kapok wax, acetated lanolin, liquid lanolin, sugarcane wax, isopropyl lanolate, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol and POE hydrogenated lanolin alcohol ether.

Examples of a hydrocarbon oil include liquid paraffin, ozocerite, squalane, pristane, paraffin, ceresin, squalene, vaseline and microcrystalline wax.

Examples of a higher alcohol include linear alcohols (e.g., lauryl alcohol, cetyl alcohol, stearyl alcohol, behenyl alcohol, myristyl alcohol, oleyl alcohol, cetostearyl alcohol); and branched alcohols (e.g., monostearyl glyceryl ether (batyl alcohol), 2-decyl tetradecynol, lanolin alcohol, cholesterol, phytosterol, hexyldodecanol, isostearyl alcohol, octyldodecanol).

Examples of a synthetic ester oil include isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanoline acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glycerin di-2-heptyl undecanoate, trimethylolpropane tri-2-ethylhexanoate, trimethylolpropane triisostearate, pentaerythritol tetra-2-ethylhexanoate, glycerin tri-2-ethylhexanoate, glycerin trioctanoate, glycerin triisopalmitate, trimethylolpropane triisostearate, cetyl 2-ethylhexanoate, 2-ethylhexyl palmitate, glycerin trimyristate, glyceride tri-2-heptylundecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate and triethyl citrate.

Examples of a silicone oil include linear polysiloxanes (e.g., dimethylpolysiloxane, methylphenylpolysiloxane, diphenyl-polysiloxane); cyclic polysiloxanes (e.g., octamethylcyclotetrasiloxane, decamethylcyclopentasiloxane, dodecamethylcyclohexasiloxane), silicone resins forming a three-dimensional network structure, silicone rubbers, and various types of modified polysiloxanes (e.g., amino modified polysiloxane, polyether modified polysiloxane, alkyl modified polysiloxane, fluorine modified polysiloxane).

Note that, in the composition of the present invention, a polar oil having an IOB of 0.05 or more may be blended. The content of the polar oil is suitably 50 % by mass or less, preferably 30 % by mass or less, and more preferably 15 % by mass or less. If the content is beyond 50 % by mass, it tends to cause a problem in sense of use, such as greasiness.

Examples of the polar oil having an IOB of 0.05 or more include 2-ethylhexyl paramethoxycinnamate, 2-ethylhexyl, 2-cyano-3,3-diphenylacrylate, tripropylene glycol dipivalate, cetyl octanoate, trimethylolpropane tri2-ethylhexanoate, pentaerythritol tetra(2-ethylhexanoate), glyceryl tri(2-ethylhexanoate), diethylhexyl naphthalene dicarboxylate, an alkyl benzoate (having 12 to 15 carbon atoms), glycerin tri(caprylate/caprate), propylene glycol di(caprylate/caprate) and di 2-ethylhexyl succinate.

Furthermore, the composition of the present invention further contains another ultraviolet absorber in addition to the aqueous dispersion of the oil-soluble ultraviolet absorber (component a) to be blended in a water phase.

The other ultraviolet absorber is oil-soluble and dissolved in an oil phase (internal phase), and preferably absorbs ultraviolet synergistically with the UV absorber (component a) present in a water phase.

According to the invention, the other ultraviolet absorber is selected from the group consisting of 2-ethylhexyl paramethoxycinnamate, homosalate, octyl salicylate, oxybenzone, 4-t-butyl-4'-methoxydibenzoylmethane, octyl triazone, methylene bis-benzotriazolyl tetramethylbutyl phenol, 2-hydroxy-4-methoxybenzophenone, dihydroxydimethoxybenzophenone, dihydroxybenzophenone, tetrahydroxybenzophenone, hexyl diethylamino hydroxybenzoyl benzoate, 2-cyano-3,3-diphenylacrylic acid 2'-ethylhexyl ester, polysilicone-15 and drometrizole polysiloxane.

In the composition of the present invention, in addition to the aforementioned components, other components usually used in external use compositions such as cosmetics can be contained as long as it does not substantially suppress a desired effect of the present invention.

The composition of the present invention can be prepared by separately mixing components constituting an oil phase and components constituting a water phase, and adding the oil phase to the water phase, so as to emulsify the resultant mixture.

The composition of the present invention provides fresh sense of use that an oil-in-water emulsion originally has, has excellent stability at a low temperature and a high temperature and exerts an excellent ultraviolet protection ability, and thus is particularly suitable for use as oil-in-water emulsion type sunscreen cosmetics.

### Examples

The present invention will be described in more detail by way of specific examples below; however, the present invention is not limited to the following Examples. Furthermore, the contents shown in the following Examples and the like are expressed by % by mass, if not otherwise specified.

### (Examples and Comparative Examples)

Oil-in-water emulsion compositions having compositions shown in the following Table 1 were prepared. To describe more specifically, water phase components and oil phase components were each heated to 70°C to be completely dissolved, and then, the oil phase was added to the water phase and emulsified by an emulsifier to obtain compositions of each Examples..

A sample (18.87 µL) was taken from each of the compositions of Example 1 and Comparative Example 1 and uniformly applied to a surface of a film made of PMMA (5 cm x 5 cm) in a ratio of 0.75 mg/cm². After the film was allowed to stand still for 15 minutes, absorbance of each of the samples was measured by using a spectrophotometer (U-4100: manufactured by Hitachi, Ltd.). The results of the measurements are shown in Figure 1.

Separately from the above, the liquid oil contents of Example 1 (olefin oligomer, glyceryl 2-ethylhexanoate, octocrylene) were mixed in accordance with the ratio described in Table 1 to prepare an oil mixture corresponding to Example 1. To this oil mixture, bis-ethylhexyloxyphenol methoxyphenyl triazine was further added. The mixture was heated to 70°C to be completely dissolved, and thus, an oil mixture corresponding to Comparative Example 1 was prepared. The resultant mixtures were each placed in a 50 mL screw tube and cooled to 25°C. To these mixtures, a small amount of bis-ethylhexyloxyphenol methoxyphenyl triazine was added in a solid state. Each of the mixtures, after being stored at 0°C for one week, was observed by a microscope to evaluate stability of the oil phases. These results are collectively shown in Table 1, in which the case where crystals in the amount equal to or larger than the addition amount were observed is expressed as "Poor" and the case where the crystals in the amount equal to or larger than the addition amount were not observed is expressed as "Good".

**[Table 1]**

| | | Comparative Example 1 | Example 1 |
|---|---|---|---|
| | Ion exchange water | Balance | Balance |
| | Ethyl alcohol | 5.0 | 5.0 |
| | 1,3-Butylene glycol | 5.0 | 5.0 |
| | Glycerin | 5.0 | 5.0 |
| | Xanthan gum | 0.1 | 0.1 |
| Water phase | Saponite^{*1)} | 0.3 | 0.3 |
| | Triethanolamine | 0.25 | 0.25 |
| | EDTA- 3 Na₂H₂O | 0.1 | 0.1 |
| | Phenoxyethanol | 0.5 | 0.5 |
| | Aqueous dispersion of bis-ethylhexyloxyphenol *²⁾ methoxyphenyl triazine^{*2)} | - | 15.0 |
| | Glyceryl monostearate | 1.5 | 1.5 |
| | POE glyceryl isostearate | 1.5 | 1.5 |
| | Behenic acid | 1.0 | 1.0 |
| | Polyvinyl pyrrolidone/eicosene copolymer | 2.0 | 2.0 |
| | Behenyl alcohol | 1.5 | 1.5 |
| | Olefin oligomer | 5.0 | 5.0 |
| Oil phase | Glyceryl 2-ethylhexanoate | 5.0 | 5.0 |
| | Octocrylene | 10 | 10 |
| | bis-Ethylhexyloxyphenol methoxyphenyl triazine | 3.0 | - |
| | Hexyl diethylamino hydroxybenzoyl benzoate | 3.0 | 3.0 |
| | 4-tert-Butyl-4'-methoxybenzoyl methane | 3.0 | 3.0 |
| | Polysilicone-15 | 5.0 | 5.0 |
| | Fragrance | q.s. | q.s. |
| Total | | 100 | 100 |
| Stability of oil phase | | Poor | Good |

| | | | |
|---|---|---|---|
| *1) Smecton SA (manufactured by Kunimine Industries Co., Ltd.) *2) Tinosorb S Aqua (manufactured b BASF) | | | |

As is apparent from Table 1, in Comparative Example 1 where bis-ethylhexyloxyphenol methoxyphenyl triazine was blended in an oil phase, stability was low and crystals were formed at a low temperature (0°C). Furthermore, from the results shown in Figure 1, it was found that the composition (Example 1) in which bis-ethylhexyloxyphenol methoxyphenyl triazine was blended in a water phase in the form of an aqueous dispersion exhibits more excellent ultraviolet absorption ability than Comparative Example 1 where bis-ethylhexyloxyphenol methoxyphenyl triazine was blended in an oil phase.

Oil-in-water emulsion compositions having compositions shown in the following Table 2 were prepared. To describe more specifically, water phase components and oil phase components were each heated to 70°C to be completely dissolved, and then, the oil phase was added to the water phase and emulsified by an emulsifier to obtain compositions of each Examples. Each of the obtained compositions was placed in a 50 mL screw tube and stored at 60°C for one week. Thereafter, emulsion stability was evaluated from the appearance. These results are collectively shown in Table 2 in which the case where a single phase state was maintained is expressed as "Good" and the case where the phase was separated is expressed as "Poor".

**[Table 2]**

| | | Comparative Example 2 | Example 2 | Example 3 | Example 4 |
|---|---|---|---|---|---|
| | Ion exchange water | Balance | Balance | Balance | Balance |
| | Ethyl alcohol | 5.0 | 5.0 | 5.0 | 5.0 |
| | Dipropylene glycol | 5.0 | 5.0 | 5.0 | 5.0 |
| | Glycerin | 5.0 | 5.0 | 5.0 | 5.0 |
| | Xanthan gum | 0.1 | 0.1 | 0.1 | 0.1 |
| Water phase | Carbomer | 0.1 | - | - | - |
| | Bentonite^{*1)} | - | 0.1 | 1.0 | 2.0 |
| | Triethanolamine | 0.25 | 0.25 | 0.25 | 0.25 |
| | EDTA-3Na2H₂O | 0.1 | 0.1 | 0.1 | 0.1 |
| | Phenoxyethanol | 0.5 | 0.5 | 0.5 | 0.5 |
| | Aqueous dispersion of bis-ethylhexyloxyphenol methoxyphenyl triazine ^{*2}) | 10.0 | 10.0 | 10.0 | 10.0 |
| | Glyceryl monostearate | 1.5 | 1.5 | 1.5 | 1.5 |
| | POE glyceryl isostearate | 1.5 | 1.5 | 1.5 | 1.5 |
| | POE glycerin monoisostearate | 0.5 | 0.5 | 0.5 | 0.5 |
| | Stearic acid | 1.0 | 1.0 | 1.0 | 1.0 |
| | Polyvinyl pyrrolidone/eicosene copolymer | 2.0 | 2.0 | 2.0 | 2.0 |
| | Batyl alcohol | 0. 5 | 0. 5 | 0.5 | 0.5 |
| Oil phase | Behenyl alcohol | 1.0 | 1.0 | 1.0 | 1.0 |
| | Isononyl isononanoate | 5.0 | 5.0 | 5,0 | 5.0 |
| | Glyceryl 2-ethylhexanoate | 5.0 | 5.0 | 5.0 | 5.0 |
| | 2-Ethylhexyl paramethoxycinnamate | 5.0 | 5.0 | 5.0 | 5.0 |
| | 2-Cyano-3,3-diphenylacrylic acid 2'-ethylhexyl ester | 5.0 | 5.0 | 5.0 | 5.0 |
| | Hexyl diethylamino hydroxybenzoyl benzoate | 3.0 | 3.0 | 3.0 | 3.0 |
| | Fragrance | q.s. | q.s. | q.s. | q.s. |
| Total | | 100 | 100 | 100 | 100 |
| Emulsion temperature | stability at high | Poor | Good | Good | Good |

| | | | | | |
|---|---|---|---|---|---|
| *1) Kunipia (manufactured by Kunimine Industries Co., Ltd.) *2) Tinosorb S Aqua (manufactured b BASF) | | | | | |

From the results shown in Table 2, it was found that in Comparative Example 2 in which only xanthan gum and a carbomer, which are water-soluble polymers, were blended, separation occurred; whereas, in Examples 2 to 4 in which a water-swellable clay mineral (bentonite) was blended, a composition having good stability can be obtained.

Cosmetics composed of oil-in-water emulsion compositions in accordance with the following formulations were prepared.

**Formulation Example 1: Sunscreen Emulsion**

| | |
|---|---|
| Dipropylene glycol | 5 |
| Xanthan gum | 0.1 |
| Bentonite | 1 |
| Stearic acid | 0.5 |
| Palmitic acid | 0.5 |
| Polyoxyethylene glyceryl isostearate | 1 |
| Glycerin monostearate | 1 |
| Polyoxyethylene glycerin monostearate | 1 |
| Polyvinyl pyrrolidone/eicosene copolymer | 1 |
| Tripropylene glycol dineopentanoate | 5 |
| Squalane | 3 |
| Decamethyl cyclohexapentasiloxane | 4 |
| Dimethylpolysiloxane | 2 |
| 2-ethylhexyl paramethoxycinnamate | 7 |
| 2-hydroxy-4-methoxybenzophenone | 2 |
| Methylene bis-benzotriazolyl tetramethylbutyl phenol | 1 |
| Aqueous dispersion of bis-ethylhexyloxyphenol methoxyphenyl triazine | 15 |
| Sodium hexametaphosphate | 0.1 |
| Triethanolamine | q.s. |
| Antiseptic agent | q.s. |
| Pure water | balance |
| Fragrance | q.s. |

**Formulation Example 2: Sunscreen Emulsion**

| | |
|---|---|
| Glycerin | 5 |
| Carbomer | 0.3 |
| Saponite (manufactured by Kunimine Industries Co., Ltd.) | 0.5 |
| Stearic acid | 0.5 |
| Isostearic acid | 0.5 |
| Stearyl alcohol | 2 |
| Polyoxyethylene hydrogenated castor oil | 1 |
| Polyoxyethylene glycerin monostearate | 1 |
| Trimethyl siloxysilicate | 1 |
| Caprylyl methicone | 3 |
| Cetyl ethylhexanoate | 10 |
| 2-Ethylhexyl paramethoxycinnamate | 5 |
| 2-Cyano-3,3-diphenylacrylic acid 2'-ethylhexyl ester | 5 |
| Aqueous dispersion of bis-ethylhexyloxyphenol methoxyphenyl triazine | 10 |
| Octyl triazone | 2 |
| Hexyl diethylaminohydroxybenzoyl benzoate | 2 |
| Phenylbenzimidazole sulfonic acid | 1 |
| 2-glucoside ascorbate | 2 |
| EDTA-3Na | 0.1 |
| Potassium hydroxide | q.s. |
| Antiseptic agent | q.s. |
| Pure water | balance |
| Fragrance | q.s. |

**Formulation Example 3: Sunscreen Emulsion**

| | |
|---|---|
| Alcohol | 5 |
| Dipropylene glycol | 5 |
| Xanthan gum | 0.1 |
| Bentonite | 0.5 |
| Glyceryl monostearate | 1 |
| Polyoxyethylene glyceryl isostearate | 1 |
| Behenic acid | 1 |
| Behenyl alcohol | 2 |
| Trimethylsiloxy silicic acid | 1 |
| Cyclomethicone | 3 |
| Dimethicone | 2 |
| Isopropyl myristate | 5 |
| Octyl palmitate | 5 |
| Diethylhexyl succinate | 1 |
| Polyoxyethylene-polyoxypropylene glycol | 1 |
| 2-Cyano-3,3-diphenyl acrylic acid-2'-ethylhexyl este | 5 |
| Aqueous dispersion of bis-Ethylhexyloxyphenol | |
| methoxyphenyl triazine | 10 |
| Hexyl diethylaminohydroxybenzoyl benzoate | 2 |
| 4-tert-Butyl-4'-methoxydibenzoyl methane | 2 |
| Methylene bis-benzotriazolyl tetramethylbutylphenol | 2 |
| EDTA-3Na | 0.1 |
| Triethanolamine | q.s. |
| Antiseptic agent | q.s. |
| Pure water | balance |
| Fragrance | q.s. |

## Claims

1. An oil-in-water emulsion composition comprising:
(a) an aqueous dispersion of composite particles of a first oil-soluble ultraviolet absorber and an organic polymer contained in an external phase (water phase), wherein the first oil-soluble ultraviolet absorber is bis-ethylhexyloxyphenol methoxyphenyl triazine,
(b) a nonionic surfactant and a fatty acid soap,
(c) a water-swellable clay mineral selected from the group consisting of saponite and bentonite,
(d) a higher fatty acid, and
(e) a second oil-soluble ultraviolet absorber dissolved in an internal phase (oil phase), wherein the second oil-soluble ultraviolet absorber is selected from the group consisting of 2-ethylhexyl paramethoxycinnamate, homosalate, octyl salicylate, oxybenzone, 4-t-butyl-4'-methoxydibenzoylmethane, octyl triazone, methylene bis-benzotriazolyl tetramethylbutyl phenol, 2-hydroxy-4-methoxybenzophenone, dihydroxydimethoxybenzophenone, dihydroxybenzophenone, tetrahydroxybenzophenone, hexyl diethylamino hydroxybenzoyl benzoate, 2-cyano-3,3-diphenylacrylic acid 2'-ethylhexyl ester, polysilicone-15 and drometrizole polysiloxane.

2. The composition according to claim 1, wherein the second oil-soluble ultraviolet absorber is selected from the group consisting of 2-ethylhexyl paramethoxycinnamate, 4-t-butyl-4'-methoxydibenzoylmethane, octyl triazone, methylene bis-benzotriazolyl tetramethylbutyl phenol, 2-hydroxy-4-methoxybenzophenone, hexyl diethylamino hydroxybenzoyl benzoate, 2-cyano-3,3-diphenylacrylic acid 2'-ethylhexyl ester and polysilicone-15.

3. The composition according to claim 1 or 2, wherein the composition comprising 3 % by mass or less of a water-soluble polymer.

4. The composition according to any one of claims 1 to 3, wherein a content of the first oil-soluble ultraviolet absorber is 5 % by mass or less.

5. The composition according to any one of claims 1 to 4, wherein a content of the (c) water-swellable clay mineral is 0.01 to 4 % by mass.

6. The composition according to any one of claims 1 to 5, wherein the composition is a sunscreen cosmetic.

## Patentansprüche

1. Zusammensetzung in Form einer Öl-in-Wasser-Emulsion, umfassend:
(a) eine wässrige Dispersion von Verbundpartikeln eines ersten öllöslichen Ultraviolettabsorbers und eines organischen Polymers, welche in einer externen Phase (Wasserphase) enthalten sind, wobei es sich bei dem ersten öllöslichen Ultraviolettabsorber um bis-Ethylhexyloxyphenolmethoxyphenyltriazin handelt,
(b) ein nichtionisches Tensid und eine Fettsäureseife,
(c) ein wasserquellbares Tonmineral ausgewählt aus der Gruppe bestehend aus Saponit und Bentonit,
(d) eine höhere Fettsäure, und
(e) einen zweiten öllöslichen Ultraviolettabsorber, welcher in einer internen Phase (Ölphase) gelöst ist, wobei der zweite öllösliche Ultraviolettabsorber aus der Gruppe bestehend aus 2-Ethylhexylparamethoxycinnamat, Homosalat, Octylsalicylat, Oxybenzon, 4-t-Butyl-4'-methoxydibenzoylmethan, Octyltriazon, Methylen-bisbenzotriazolyltetramethylbutylphenol, 2-Hydroxy-4-methoxybenzophenon, Dihydroxydimethoxybenzophenon, Dihydroxybenzophenon, Tetrahydroxybenzophenon, Hexyldiethylaminohydroxybenzoylbenzoat, 2-Cyano-3,3-diphenylacrylsäure-2'-ethylhexylester, Polysilikon-15 und Drometrizolpolysiloxan ausgewählt ist.

2. Zusammensetzung nach Anspruch 1, wobei der zweite öllösliche Ultraviolettabsorber aus der Gruppe bestehend aus 2-Ethylhexylparamethoxycinnamat, 4-t-Butyl-4'-methoxydibenzoylmethan, Octyltriazon, Methylen-bisbenzotriazolyltetramethylbutylphenol, 2-Hydroxy-4-methoxybenzophenon, Hexyldiethylaminohydroxybenzoylbenzoat, 2-Cyano-3,3-diphenylacrylsäure-2'-ethylhexylester und Polysilikon-15 ausgewählt ist.

3. Zusammensetzung nach Anspruch 1 oder 2, wobei die Zusammensetzung 3 Masse% oder weniger an einem wasserlöslichen Polymer umfasst.

4. Zusammensetzung nach einem der Ansprüche 1 bis 3, wobei der Gehalt an dem ersten öllöslichen Ultraviolettabsorber 5 Masse% oder weniger beträgt.

5. Zusammensetzung nach einem der Ansprüche 1 bis 4, wobei der Gehalt an dem (c) wasserquellbaren Tonmineral 0.01 bis 4 Masse% beträgt.

6. Zusammensetzung nach einem der Ansprüche 1 bis 5, wobei es sich bei der Zusammensetzung um ein Sonnenschutzkosmetikum handelt.

## Revendications

1. Composition d'émulsion huile-dans-eau, comprenant :
(a) une dispersion aqueuse de particules composites d'un premier absorbeur d'ultraviolet oléosoluble et d'un polymère organique contenues dans une phase externe (phase aqueuse), dans laquelle le premier absorbeur d'ultraviolet oléosoluble est la bis-éthylhexyloxyphénol méthoxyphényl triazine,
(b) un agent tensioactif non ionique et un savon d'acide gras,
(c) un minéral argileux gonflable dans l'eau choisi dans le groupe constitué par la saponite et la bentonite,
(d) un acide gras supérieur, et
(e) un second absorbeur d'ultraviolet oléosoluble dissous dans une phase interne (phase huileuse), lequel second absorbeur d'ultraviolet oléosoluble est choisi dans le groupe constitué par le paraméthoxycinnamate de 2-éthylhexyle, un homosalate, le salicylate d'octyle, l'oxybenzone, le 4-t-butyl-4'-méthoxydibenzoylméthane, l'octyl triazone, le méthylène bis-benzotriazolyl tétraméthylbutyl phénol, la 2-hydroxy-4-méthoxy-benzophénone, la dihydroxydiméthoxybenzophénone, la dihydroxybenzophénone, la tétrahydroxybenzophénone, le benzoate d'hexyl diéthylamino hydroxybenzoyle, l'ester 2'-éthylhexylique d'acide 2-cyano-3,3-diphényl-acrylique, la polysilicone-15 et un drométrizole polysiloxane.

2. Composition selon la revendication 1, dans laquelle le second absorbeur ultraviolet oléosoluble est choisi dans le groupe constitué par le paraméthoxycinnamate de 2-éthylhexyle, le 4-t-butyl-4'-méthoxydibenzoylméthane, l'octyl triazone, le méthylène bis-benzotriazolyl tétraméthylbutyl phénol, la 2-hydroxy-4-méthoxybenzophénone, le benzoate d'hexyl diéthylamino hydroxybenzoyle, l'ester 2'-éthylhexylique d'acide 2-cyano-3,3-diphénylacrylique et la polysilicone-15.

3. Composition selon la revendication 1 ou 2, laquelle composition comprend 3 % en masse ou moins d'un polymère hydrosoluble.

4. Composition selon l'une quelconque des revendications 1 à 3, dans laquelle une teneur en le premier absorbeur d'ultraviolet oléosoluble est de 5 % en masse ou moins.

5. Composition selon l'une quelconque des revendications 1 à 4, dans laquelle une teneur en le (c) minéral argileux gonflable dans l'eau est de 0,01 à 4 % en masse.

6. Composition selon l'une quelconque des revendications 1 à 5, laquelle composition est un produit cosmétique écran solaire.
